# FASCICULE DE BREVET EUROPEEN

(11) **EP 0 741 564 B1**
(45) Date de publication et mention de la délivrance du brevet: **05.04.2006**
(21) Numéro de dépôt: 95934170.2
(22) Date de dépôt: 05.10.1995
(51) Int. Cl.: A61K 9/00

(54) **NOUVEAU PROCEDE DE FABRICATION DE SUPPOSITOIRES**
NEUES VERFAHREN ZUM HERSTELLEN VON SUPPOSITORIEN
NOVEL METHOD OF THE MANUFACTURE OF SUPPOSITORIES

(30) Priorité: 05.10.1994 FR 9411913
(43) Date de publication de la demande: 13.11.1996
(73) Titulaire: TECHNIPHARMA, 98014 Monaco Cédex (MC)
(72) Inventeur: SIRITO, Alain, MC-98014 Monaco Cédex (MC); NOTE-SIMONNARD, Axelle, MC-98014 Monaco Cédex (MC)
(74) Mandataire: Burtin, Jean-François
(86) Numéro de dépôt international: PCT/FR1995/001294
(87) Numéro de publication internationale: WO 1996/010987

(56) Documents cités:
- EP-A- 0 088 394
- FR-A- 788 198
- FR-M- 5 391
- CHEMICAL ABSTRACTS, vol. 94, no. 10, 9 Mars 1981 Columbus, Ohio, US; abstract no. 71519, & JP,A,55 136 215 (INSTITUTE FOR PRODUCTION AND DEVELOPMENT OF SCIENCE,JP) 23 Octobre 1980
- CHEMICAL ABSTRACTS, vol. 118, no. 24, 14 Juin 1993 Columbus, Ohio, US; abstract no. 240769, & CHEM. PHARM. BULL., vol. 41, no. 2, 1993 pages 351-356, T.HAKATA ET AL. 'FORMULATION STUDIES OF EFFERVESCENT SUPPOSITORIES.II.EFFECTS OF BASES AND ADDITIVES ON RELEASE OF CARBON DIOXIDE FROM EFFERVESCENT SUPPOSITORIES.'

## Description

La présente invention se rapporte au domaine de la pharmacotechnie.

Elle a plus particulièrement pour objet un nouveau procédé de préparation de suppositoires et notamment de suppositoires à action laxative.

Elle a spécifiquement pour objet un procédé de préparation de suppositoires à action laxative susceptibles de provoquer un dégagement de gaz carbonique par réaction chimique de principes actifs au contact de l'humidité présente dans l'ampoule rectale.

On connaît, par le brevet français n° 788.198 (Waldenmeyer J.G), un procédé qui offre la possibilité de faire dégager de l'acide carbonique à l'état naissant sous l'action de l'humidité ou autre cause et dans lequel, les matières premières dégageant par leur mélange cet acide, sont enrobées une à une dans une substance grasse qui les protège contre une décomposition prématurée et qui leur permet; cependant, d'être atteintes au moment de l'emploi par l'action catalytique d'un agent hydrophile.

On peut déduire, à la lecture de ce brevet, qu'il pourrait s'agir d'un procédé de préparation de suppositoires dégageant de l'acide carbonique mais rien n'est dit à ce sujet et seul l'emploi d'un corps gras comme le beurre de cacao peut laisser soupçonner une telle utilisation.

Par la suite, un médicament utilisant ce procédé est apparu sur le marché. Il est formé d'un suppositoire contenant un mélange effervescent de tartrate acide de potassium et de bicarbonate de sodium susceptible de libérer, en milieu humide, environ de 50 ml à 100 ml de gaz carbonique au niveau du rectum.

Le problème créé par la mise au point de ce médicament réside dans la difficulté de réaliser un produit qui ne réagisse pas prématurément à la fabrication ou peu de temps après la fabrication, tout en gardant intactes les propriétés d'effervescence désirées.

On se trouve donc confronté à un double problème. En effet, les principes actifs sont séparés l'un de l'autre par une barrière notamment lipophile, absolument étanche.

Grâce à celle-ci, le médicament est en mesure de se garder parfaitement mais en revanche, les principes actifs ne pourront pas réagir l'un avec l'autre, de telle sorte qu'un tel médicament est pratiquement inactif.

Au contraire, si les principes actifs ne sont pas englobés dans une matrice imperméable, ils sont susceptibles de réagir entre eux prématurément dans l'emballage de conditionnement et surtout, ils sont susceptibles de réagir entre eux au niveau de l'ampoule rectale d'une manière trop violente et de déclancher des distensions trop importantes de l'ampoule rectale.

Il était donc important de trouver un enrobage suffisamment isolant pour empêcher les constituants du mélange effervescent de réagir précocément les uns avec les autres mais aussi apte à amener, au contact d'une muqueuse plus ou moins humide, comme l'ampoule rectale, un dégagement régulier, constant et progressif de gaz carbonique.

Cet objectif peut être considéré comme atteint avec le procédé conforme à l'invention.

Dans ce procédé, les matières grasses sont mises à fondre séparement, on laisse refroidir le mélange à la température désirée et dans la masse fluide résultant de la fusion, on disperse de la lécithine végétale puis on incorpore un agent opacifiant minéral, on verse ensuite successivement, sous agitation, le tartrate acide de potassium puis sous plus forte agitation, le bicarbonate de sodium, ces deux constituants présentant une granulométrie particulière, puis on poursuit l'agitation jusqu'à parfaite homogénéité avant de procéder au soutirage de la suspension fluide dans les alvéoles de suppositoires.

Après refroidissement, on obtient des suppositoires de forme tronc-conique régulière dont la composition est homogène et dont la conservation, attestée par le volume du dégagement de gaz carbonique, est assurée pendant deux ans au moins.

On voit donc clairement que le problème technique à résoudre est sensiblement différent de celui envisagé par le procédé Waldenmeyer. A cette époque, les matières grasses constituaient un enrobage étanche qui protégeait complètement, et même trop complètement, contre l'action catalytique d'un agent hydrophile et il était nécessaire d'y incorporer un produit facilitant le passage des milieux aqueux.

Au contraire, dans la technique présente, les excipients pour suppositoires sont faits de stéarates de poly éthylèneglycol ou de triglycérides d'acides gras à chaîne moyenne qui sont des substances à la fois lipophiles et hydrophiles, de telle sorte qu'il faut protéger les substances réactives du mélange par un écran protecteur inerte et non plus par des corps lipoïdes.

Dans un mode d'exécution préféré du procédé selon l'invention, les matières grasses qui servent de support pour la réalisation de suppositoires sont les triglycérides d'acides gras à chaîne moyenne vendus sous la dénomination Novata BD de la Société HENKEL. On peut également utiliser ceux vendus sous la dénomination Estaram H15 par la société UNICHEMA ou ceux vendus sous la dénomination Suppocire AM par la Société GATTEFOSSE ou encore ceux vendus sous la dénomination Witepsol H15 par la société HÜLS. Ces triglycérides présentent une zone de fusion allant de 35 à 39°C.

La lécithine végétale est une lécithine de soja et notamment la qualité vendue sous la dénomination Topcithin 50 par la Société LUCAS MEYER ou encore celle vendue sous la dénomination MC Thin AF1 par la même Société. La lécithine permet d'éviter ou de réduire l'épaississement de la masse avant coulée et s'utilise dans les cas de principes actifs solides qui se solubilisent partiellement ou, surtout, dans les cas de principes actifs pulvérulents. Selon les cas, la lécithine se dissout dans la matière grasse ou, au contraire, reste en suspension.

L'agent opacifiant est un silicate naturel ou artificiel comme le talc ou le silicate de magnésium, ou un stéarate de métal alcalino-terreux comme le stéarate de calcium ou le stéarate de magnésium, ou bien encore un dérivé du titane comme le dioxyde de titane, ou **un titanate insoluble comme le** titanate de baryum.

La granulométrie des constituants du mélange effervescent (tartrate acide de potassium et bicarbonate de sodium) est réglée de façon à ce que les poudres présentent une grande finesse et se répartissent d'une manière parfaitement homogène sans décanter sous agitation au fur et à mesure de la préparation.

La température des suppositoires, après coulée dans les alvéoles, est progressivement diminuée par refroidissement jusqu'à complète solidification.

Les suppositoires ainsi réalisés possèdent un délai de peremption de deux ans, ce qui garantit leur conservation pendant une période au moins égale.

L'exemple suivant illustre l'invention sans toutefois la limiter.

### EXEMPLE I

### Préparation de suppositoires effervescents

Dans une cuve en acier inoxydable, on introduit 36,7 kg de glycérides hémi-synthétiques solides que l'on fait fondre à une température comprise entre 35 et 39°C. On ajoute, ensuite, sous agitation, 4,2 kg de lécithine de soja. Puis on incorpore progressivement, toujours sous agitation, 2,1 kg de talc. Après avoir réalisé l'homogénéité de la suspension, on ajoute par petites portions et sous forte agitation, 23 kg de tartrate acide de potassium puis 14 kg de bicarbonate de sodium.

On laisse mélanger sous agitation pendant 10 minutes puis, en maintenant la température, on soutire la suspension et on remplit ainsi les alvéoles que l'on passe dans une enceinte de refroidissement jusqu'à complète solidification.

### Détermination de l'effervescence des suppositoires:

Principe :

### Contrôle de l'effervescence par mesure de dégagement de gaz carbonique à 37°C

### Technique :

On introduit rapidement le suppositoire dans une éprouvette A (100 ml) remplie d'eau à 37°C et on ferme celle-ci avec un bouchon en verre fritté (porosité 2) muni d'un joint en caoutchouc, en veillant à ne pas emprisonner de bulle d'air.

On plonge l'éprouvette A dans une seconde éprouvette B (250 ml) remplie d'eau à 37°C et contenant un barreau magnétique. L'éprouvette B est placée dans un bain-marie à environ 40°C, disposé sur un agitateur magnétique chauffant, de façon à maintenir une température constante (37° ± 1 °C).

On maintient l'agitation magnétique et le chauffage pendant tout le dégagement gazeux. On lit le volume de gaz carbonique sur l'éprouvette A.

Essais dé stabilité des suppositoires effervescents et dosage du gaz carbonique libéré.

Les contrôles de l'effervescence ont été effectués sur plusieurs lots de fabrication réalisés à trois périodes différentes.

Ces contrôles ont été réalisés :
- sur un même blister provenant du début, du milieu ou de la fin de la fabrication
- et sur plusieurs blisters provenant du début, du milieu et de la fin de la fabrication.

Ces contrôles ont permis de mettre en évidence :
- sur un même blister pris à un moment donné de la fabrication (début, milieu ou fin), l'écart maximal de volume de gaz carbonique libéré et donc l'homogénéité intra-blister
- sur plusieurs blisters pris à des moments différents de la fabrication (début, milieu ou fin), l'écart maximal de volume de gaz carbonique libéré et donc l'homogénéité inter-blister.
- le volume moyen de gaz carbonique libéré au début, au milieu et à la fin de la fabrication
- le volume global moyen de gaz carbonique libéré.

Tous ces contrôles ont été effectués dans une éprouvette de 250 ml.

Les conclusions sont les suivantes :

Le dégagement gazeux moyen se situe entre 50 et 100 ml.

Il n'y a pas de baisse significative de volume de gaz carbonique dégagé entre un lot au temps O et un lot préparé deux ans auparavant.

Les meilleurs dégagements gazeux se situent, dans la plupart des cas, au milieu de la fabrication et les plus mauvais dégagements se situent, dans la plupart des cas, en fin de fabrication.

Pour plus de précision, les contrôles effectués ont donné les résultats suivants :

| **Fabrication Effervescence** | **début** | **10 heures** | **12 heures** | **14 heures** | **fin** |
|---|---|---|---|---|---|
| | Lot 1 | Lot 2 | Lot 3 | Lot 4 | Lot 5 |
| Contrôle n°1 | 94 ml | 80 ml | 80 ml | 76 ml | 76 ml |
| Contrôle n°2 | 91 ml | 84 ml | 86 ml | 82 ml | 80 ml |
| Contrôle n°3 | 93 ml | 73 ml | 84 ml | 80 ml | 78 ml |

En conclusion, les variations constatées tiennent compte à la fois de la difficulté d'obtenir une préparation absolument homogène et aussi de la sensibilité incertaine des méthodes d'appréciation du volume de dégagement gazeux en raison de la solubilité du gaz carbonique dans l'eau.

## Revendications

1. Procédé de préparation de suppositoires effervescents susceptibles de provoquer un dégagement de gaz carbonique régulier, constant et progressif, par réaction chimique du mélange réactif au contact de l'humidité,
**caractérisé en ce qu'**
on met à fondre séparément les matières grasses, qu'on laisse refroidir le mélange à la température désirée, qu'on incorpore, sous agitation, dans le mélange, la lécithine végétale, puis un agent opacifiant minéral sélectionné entre un silicate naturel ou artificiel, un stéarate de métal alcalino-terreux, le stéarate de magnésium ou bien encore le dioxyde de titane ou un titanate insoluble, puis on verse ensuite successivement les deux éléments qui génèrent du gaz carbonique, on poursuit l'agitation jusqu'à ce que le mélange soit parfaitement homogène et on procède au soutirage de la suspension fluide dans les alvéoles de suppositoires.

2. Procédé de préparation de suppositoires effervescents selon la revendication 1,
**caractérisé en ce que**
la matière grasse fond entre 35 et 39°C.

3. Procédé de préparation de suppositoires effervescents selon la revendication 1,
**caractérisé en ce que**
les éléments qui génèrent du gaz carbonique sont respectivement le tartrate acide de potassium et le bicarbonate de sodium.

4. Procédé selon les revendications 1 à 3,
**caractérisé en ce que**
les deux constituants qui génèrent du gaz carbonique présentent une granulométrie particulière.

## Patentansprüche

1. Verfahren für die Bereitung von ausbrausenden Zäpfchen die eine gleichmässige, beständige, fortschreitende Freisetzung von Kohlenstoffdioxid beim Fühlung mit Feuchtigkeit, durch eine chemische Vorgang der reagierende Mischung bewirken können,
**dadurch gekennzeichnet,**
**dass** man zum Schmelzen einzeln die Fettstoffe auggesetzt, man die Mischung bei gewünschte Temperatur abkühlen lässt, dass man unter Umrührung in der Mischung ein pflanzische Lezithin beimengt, danach eine anorganisches Trübungsmittel das unter ein naturalen oder kunstlischen Silikat, einen Erd-alkali Metall Silikat, Magnesium Silikat oder Titanium Dioxid oder einen unlösslichen Titanate gewählt wird, danach man nach und nach die beide Bestandteile die Kohlenstoff Dioxid erzeugen, man die Ruhrung fortgehen läszt bis die Mischung perfekt.gleichmässige wird, und man die Abfüllung der dunnflussig Schlämmung in der Zelle von Zäpfchen, verfährt.

2. Verfahren für Bereitung von aufbrausenden Zäpfchen nach Anspruch 1,
**dadurch gekennzeichnet,**
**dass** die Fettstoffe zwischen 35 und 39° C schmilzt.

3. Verfahren für Bereitung von aufbrausenden Zäpfchen nach Anspruch 1,
**dadurch gekennzeichnet,**
**dass** die Bestandteile die Kohlenstoff Dioxid erzeugen, jeweils Potassium Hydrogeno-weinsteinsäure und Sodium HydrogenoCarbonat sind.

4. Verfahren nach Ansprüche 1 bis 3,
**dadurch gekennzeichnet,**
**dass** die beide Bestandteile die Kohlenstoff Dioxid erzeugen, eine bestimmte Durchmessergrosse aufweisen.

## Claims

1. A process for preparing effervescent rectal suppositories, able to cause a regular, constant and progressive evolution of carbon dioxide through a chemical reaction of a reactive mixture in the contact of wetness
**characterized in that**
fatty materials are melted separately, then the mixture is let to cool down to the desired temperature, that they are incorporated under mixing in the mixture the vegetal lecithin then a mineral opacifying agent selected among a natural or artificial silicate, an earth alkali metal silicate, magnesium stearate or else titanium dioxide or an insoluble titanate, that one pours thereafter successively the two ingredients which generate carbon dioxide, the mixing is then carried on until the mixture became perfectly homogeneous, and the decanting of the fluid suspension into the alveols of suppositories, is carried out.

2. A process for preparing the effervescent suppositories according to claim 1,
**characterized in that**
the fatty material melts between 35 to 39° C.

3. A process for preparing effervescent suppositories according to claim 1,
**characterized in that**
the ingredients which generate carbon dioxide are respectiveley potassium acid tartarate and sodium bicarbonate.

4. A process according to claims 1 to 3,
**characterized in that**
the two ingredients which generate carbon dioxid shows a definit granulometry.
